# EUROPEAN PATENT SPECIFICATION

(11) **EP 1 535 640 B1**
(45) Date of publication and mention of the grant of the patent: **25.01.2012**
(21) Application number: 03257489.9
(22) Date of filing: 27.11.2003
(51) Int. Cl.: A61M 5/32, A61M 5/46, A61M 5/00

(54) **A safety needle**
Sicherheitsspritze
Seringue de sécurité

(43) Date of publication of application: 01.06.2005
(73) Proprietor: Salvus Technology Limited, Stradbroke Suffolk IP21 5HS (GB)
(72) Inventor: Weston, Terence Edward, Swannington, Norfolk NR9 5NP (GB); Emmott, Douglas Arthur, Ipswitch, Suffolk, IP6 9LR (GB); Weston, Judith Mary, Swannington, Norfolk NR9 5NP (GB)
(74) Representative: Gillard, Richard Edward

(56) References cited:
- EP-A- 0 467 173
- EP-A- 1 252 907
- WO-A-02/089878
- WO-A-03/066141
- US-A- 3 677 245
- US-A- 4 911 693
- US-A- 4 927 019
- US-A- 5 104 384
- US-A- 5 242 401
- US-A1- 2003 144 633
- US-B1- 6 203 529
- US-B1- 6 331 174
- US-B1- 6 398 762

## Description

This invention relates to a safety needle and in particular to safety needle pack.

Needle stick injuries carry a significant risk of spreading infection, such as HIV and hepatitis, and are commonplace among healthcare workers. The USA has led the way in introducing legislation that obliges healthcare providers to use the safest devices when giving injections, intravenous drug administration and similar invasive procedures. Other countries are following and, even without legislation, the ever-present risk of litigation has alerted pharmaceutical companies and health authorities to seek suitable safe devices.

As a result of the heightened awareness of needle stick injuries, there have been a large number of inventions addressing this issue, some more successful than others. Most take the form of a protective sleeve which covers the needle tip after the injection has been given, or means for retracting the needle rapidly into the syringe barrel. Such a safety needles are exemplified by US 4,911,693, US 4,813,940 and US 5,104,384.

WO03/066141 discloses a safety shield system for a needle cannula of a pen needle injector or similar device, wherein a safety shield may be retracted from a first position enclosing the needle cannula to a second position exposing the needle cannula for injection. The safety shield system permits retraction of the safety shield during use, but extends the shield enclosing the needle cannula in a locked position following use. The end of the needle cannula is never exposed.

A common requirement for drug administration is to draw the drug from a vial or bulk container, or first reconstitute a lyophilised drug, and then draw up the required volume of drug from the reconstitution vessel. Good practice demands that separate needles are used for such procedures, to avoid contamination and "coring" of the rubber vial closure, and from a practical viewpoint, the needle used to draw up the drug usually will be larger than the delivery needle.

Often it is required for the needle tip to be visible prior to giving the injection because frequently a small amount of drug must be aspirated to clear out trapped air, and to correct the volume after drawing up the drug from a bulk container. Also, two of the most common types of injection given arc intradermal and intravenous, and both require that the needle tip be visible to ensure that the tip is inserted into the tissues or vein bevel-side up.

It is particularly problematic to provide a low-cost needle which is able to prevent needle stick injuries, whilst allowing the tip of the needle to be visible during injection. At present there arc no such devices on the market. There is therefore a need for a low cost safety needle which prevents needle stick injuries in healthcare workers but allows the tip of the needle to be exposed.

Accordingly, the present invention provides a safety needle comprising a hollow needle having a tip, a needle hub surrounding the hollow needle, a slidable sleeve slidably mounted on the needle hub and a pack surrounding the hollow needle, needle hub and slidable sleeve, the needle hub, the slidable sleeve and the pack each having a receiving end which is distal to the tip of the needle and an injection end which is proximal to the tip of the needle, wherein the receiving end of the needle hub is suitable for connection to an injection device and the slidable sleeve is adapted to slide in the direction of the length of the needle between an extended position in which the tip of the needle is located inside the slidable sleeve and a retracted position in which the tip of the needle projects from the slidable sleeve, via an intermediate position between the extended position and the retracted position in which the tip of the needle projects partially from the slidable sleeve, such that, in use, the slidable sleeve is moved in to the intermediate position for injection in to a patient and then as the needle is inserted into a patient, the slidable sleeve is caused to move into the retracted position, wherein the needle hub (7) has an outer surface (18) which is adapted to deflect the slidable sleeve (15) as, in use, the needle (3) is inserted into the patient, and the slidable sleeve (5) bears resiliently on the outer surface (18) of the needle hub (7) such that, in use, a restoring force is generated within the slidable sleeve (5) as the slidable sleeve (5) is caused to move towards the receiving end of the needle hub (7) and into the retracted position such that on removal of the needle from the patient the restoring force causes the slidable sleeve to move towards the injection end of the needle hub and into the extended position, the safety needle further comprising a locking mechanism capable of retaining the slidable sleeve in an extended position after removal of the needle from the patient, and wherein the pack is releasably mounted on the needle hub and slidable sleeve such that the injection end of the pack covers at least the injection end of needle and the receiving end of the pack has an open portion to expose the receiving end of the needle hub, and by causing the pack to be moved in a direction towards the receiving end of the needle hub, the pack engages with the slidable sleeve which is retracted from the extended position to the intermediate position.

This safety needle pack protects the user from needle stick injuries while the safety accessory, namely the needle hub and the slidable sleeve, arc primed for use. By priming the safety accessory for use, the slidable sleeve is caused to move in to an intermediate position between the extended and retracted positions such that the needle bevel is exposed.

The present invention also provides a method for priming an injection device comprising the steps of inserting an injection device in to the receiving end of the needle hub of the safety needle as claimed in any preceding claim, moving the pack towards the injection device such that the slidable sleeve moves in to the intermediate position, and removing the pack.

The present invention further provides a method for injecting a patient using the safety needle as defined above.

The present invention will now be described with reference to the following drawings, in which:
Fig. 1 shows a safety needle attached to a syringe without the pack;
Fig. 2 shows a centre line cross-section through the safety needle attached to a syringe;
Fig. 3 shows the slidable sleeve partly retracted to expose the hollow needle;
Fig. 4 shows a safety needle with the slidable sleeve in an extended and locked position;
Fig. 5 shows the detent mechanism;
Fig. 6 shows the receiving end of the slidable sleeve showing the cantilever arms;
Fig. 7 shows the barrel of a typical glass syringe used commonly for pre-filling with a drug, assembled with a safety needle;
Fig. 8 shows an integral syringe barrel and needle hub;
Fig. 9 shows a safety needle as assembled by the manufacturer, prior to inserting it into its pack;
Fig. 10 is a centre-line section of the safety needle in its pack;
Fig. 11 shows how the pack is used to hold the safety needle when connecting it to a syringe;
Fig. 12 shows the safety needle after removing the pack, and ready to use;
Fig. 12a shows an alternative shroud to increase the protection against needle stick injuries, whilst allowing the needle bevel to be seen immediately prior to giving the injection.
Fig. 13 shows the safety needle being used to inject medicament;
Fig. 14 shows the safety needle after the injection with the protective slidable sleeve locked in a forward position to cover the needle tip; and
Fig. 14a shows a centre-line section through the safety needle, rotated to show the part of the locking mechanism.

In the drawings, like parts are given the same reference numerals.

The applicants have found that the process of exposing the tip of the needle can, in itself lead to needle stick injuries. This is particularly undesirable where the user is injecting cytotoxic agents, for example during cancer chemotherapy. The safety needle pack of the present invention avoids such injuries by providing a pack which shields the user from the needle tip whilst the tip of the needle is being exposed, at least partially. By at least partially, means that the tip of the needle is not exposed to the extent that it is exposed when the user injects the needle through the skin of the patient but is exposed sufficiently for the user to see the bevel of the needle, in order to guide the tip in to a specific part of the patient or to aspirate air or expel any excess drug. When the needle tip is at least partially exposed the arrangement of the slidable sleeve and the needle hub hold the slidable sleeve in this intermediate position.

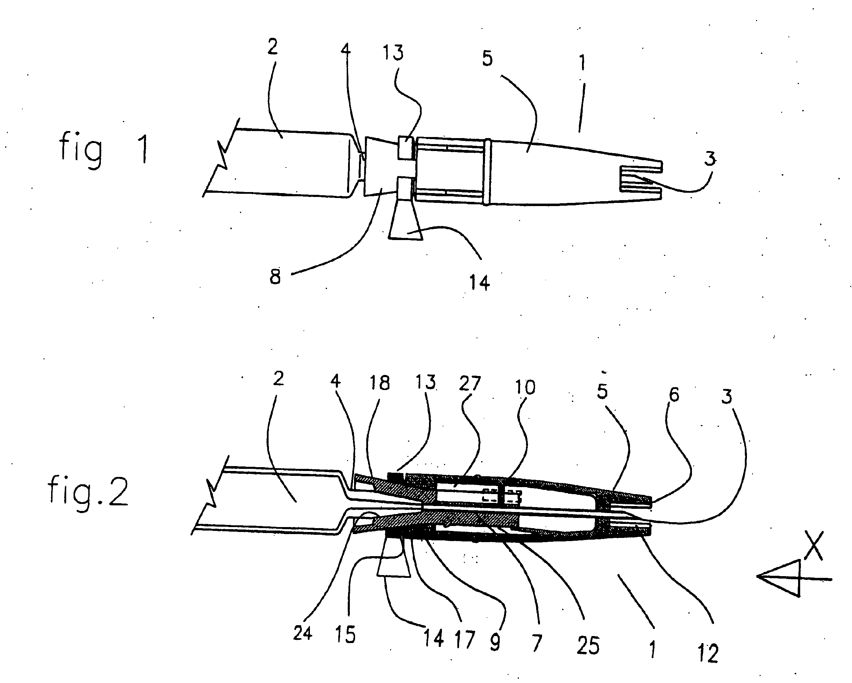

Figs. 1-8 exemplify a safety needle for preventing needle stick injuries which is suitable for use with the pack of the present invention. Fig. 1 shows the general arrangement of a safety needle 1 (i.e. the safety needle accessory and the needle 3) of the type which may be used with the present invention as fitted to a male Luer taper connector 4 of syringe 2, with the end of the hollow (e.g. hypodermic) needle 3 enclosed by the slidable sleeve 5. The slidable sleeve 5 is prevented from longitudinal movement on the needle hub 7 by a locking ring 13, which may be removed by pulling on the tab 14. The removable locking ring may be connected to the needle hub and/or the slidable sleeve. Although embodied in the Figs. as cylindrical, the cylindrical shape of the needle hub and slidable sleeve may be replaced by triangular, rectangular or other shapes to suit the application.

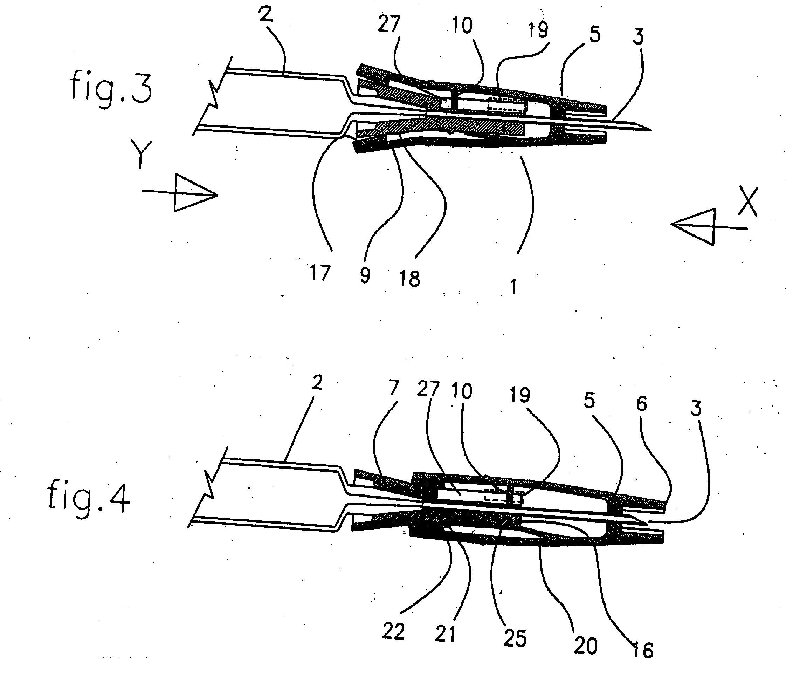

Fig. 2 is a cross-section through the axis of the safety needle 1. The needle hub 7 is cylindrical and terminates at the end which receives the syringe with a conical section 8, and is moulded onto the needle 3. The conical section 8 has an inner female Luer cone 24 which is shown frictionally attached to the male Luer cone 4 of syringe 2 (the Luer system for attaching the needle to the syringe has two main forms, that is a taper friction fit and a screw thread and both are included in the present invention). The cylindrical slidable sleeve 5 shrouds the needle 3 and the needle hub 7, and is freely sliding on and guided by the needle hub 7. At the receiving end (i.e. the syringe end) of slidable sleeve 5, there are four cantilever arms 9 which bear resiliently upon the surface 18 of the conical section 8. The slidable sleeve 5 is free to slide on the needle hub 7, but is temporarily prevented from doing so by the locking ring 13. Locking ring 13 is moulded integrally with the slidable sleeve 5 by a frangible joint 15, and may be partially or wholly detached by pulling on the tab 14 to break the frangible joint 15. It is preferred that the ring 13 remains attached to the slidable sleeve 5 to reduce the number of discarded parts. In addition, the frangible joint provides a tamper-evident lock. Alternatively, the locking ring 13 may be moulded to the needle hub 7 via a suitable frangible connection. When the locking ring 15 is removed, as shown in Fig. 3 the slidable sleeve 5 may be pushed in the direction of arrow X by acting on the face 6, when it will move relative to the needle hub 7 to expose the needle 3. As the slidable sleeve 5 moves, the cantilever arms 9 are forced outwards by the surface 18 of the conical section 8. The cantilever arms 9 are resilient and the reaction force against the surface 18 produce a resultant force Y acting against arrow X, so that when the original force is removed, the slidable sleeve 5 returns to cover the tip of needle 3. Since the restoring force is provided by the slidable sleeve 5 itself, no separate spring, e.g. a helical spring, is required in this embodiment although a separate spring could be used if desired.

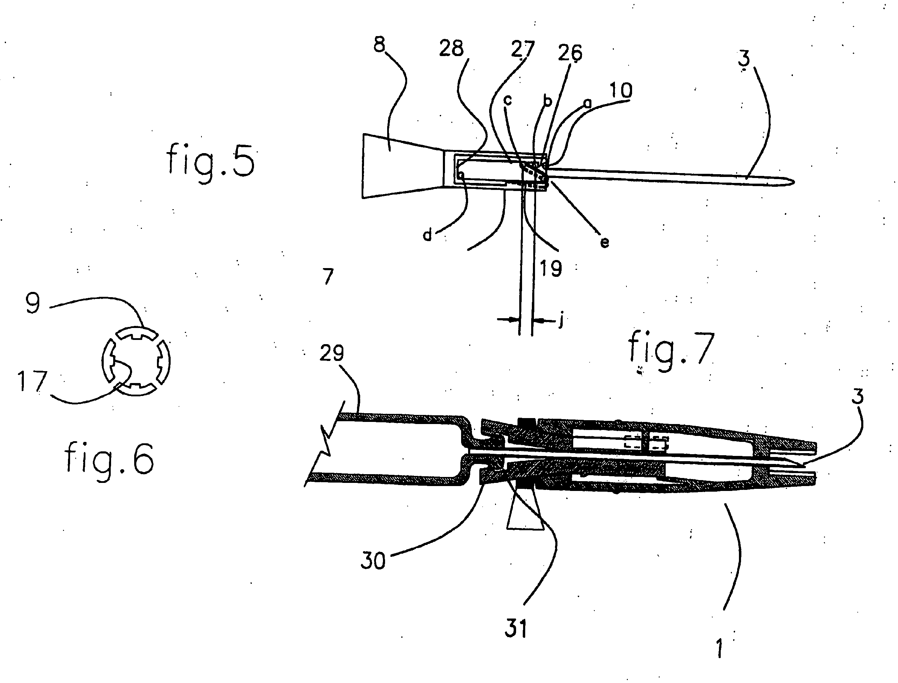

As shown in Fig. 6, the cantilever arms 9 may have pads 17 which bear onto the surface 18 and, by suitably designing the bearing surfaces of the pads 17, various spring characteristics may be obtained. Although four cantilever arms 9 are shown, any number could be employed. At least one cantilever arm 9 is required for this embodiment although 2 to 6 are preferred and particularly preferably four.

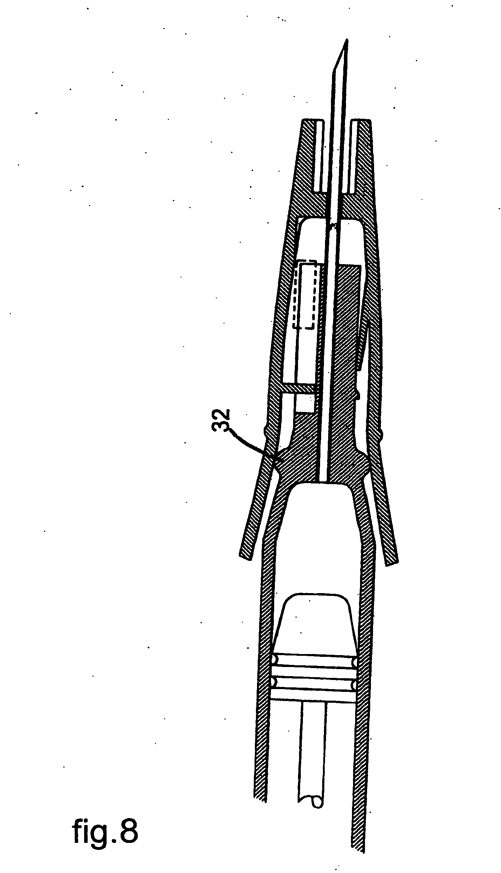

Although the surface 18 is exemplified by a conical surface, other embodiments may be used within the scope of the present invention. In the Figs. the surface is straight, i.e. substantially conical by which the applicant means sufficiently conical to generate a restoring force, however, the surface need not be straight as shown, but may be curved to give a more linear return rate. Thus the force Y could be substantially constant over a reasonable working stroke of the slidable sleeve 5. In addition, the whole surface 18 of the receiving end of the needle hub 17 need not be conical. In fact just one tapered section, e.g. a tapered ridge, would be sufficient. The tapered section does not have to project from the surface of the needle hub 7. The tapered section could also descend into the wall of the needle hub, i.e. a tapered detent rather than a tapered ridge. Also, as described below with reference to Fig. 8, provided the slidable sleeve 5 is suitably configured, a projection in the surface 18 of the needle hub 7 will suffice.

These different arrangements provide a great deal of design flexibility in the safety needle accessory of the present invention. For example, the linearity of the return rate may be varied depending on the particular requirements for a particular application.

Referring to Fig. 4, when the slidable sleeve 5 returns in direction Y, it travels slightly past its original staring position, so that the resilient pawl 20 which was depressed by the surface 25 of hub 7, snaps out to act against face 16 of hub 7. This ensures that the slidable sleeve 5 cannot be pushed back towards the syringe 2, and therefore the needle 3 is safely covered. It is preferred that there is a pre-load between the cantilever arms 9 and hub 7 to ensure that the slidable sleeve 5 is sufficiently biased in the direction of arrow Y to complete its full displacement potential.

The slidable sleeve preferably has a first extended position where the slidable sleeve is able to be moved towards the receiving end of the needle hub and a second extended position where the slidable sleeve is in a locked position. The different start and finish positions of the slidable sleeve 5 is achieved by a detent mechanism shown in Fig. 5, which is to be read in conjunction with Figs. 2, 3 and 4. After the first two or three millimetres of movement, a detent integral with the slidable sleeve "switches" so that the return of the slidable sleeve trips a latching pawl, so that the slidable sleeve returns only to the "safe" position; that is the pawl prevents the slidable sleeve from being moved towards the syringe a second time, and thus protects the tip of the needle. As part of the detent mechanism, an inside surface of the slidable sleeve has a pin 10 which engages a sprag 26 and a resilient pawl 19 attached to the needle hub 7 thereby holding the slidable sleeve in the first extended position and, in use, allowing the slidable sleeve to move into the second extended position. Pin 10 is integral or attached to the slidable sleeve 5. Sprag 26, resilient pawl 19, and recess 27 are moulded integrally with the needle hubs 7, 8, and the pin 10 extends into the recess, and allowed to move freely except where controlled by the detent and the boundaries of the recess. In the initial assembly of the safety needle, the slidable sleeve 5 is placed over the needle 3 with pin 10 proximate to the sprag 26 (formed as part of the needle hub 7) at position a (Fig. 5). As the slidable sleeve 5 is moved further, the pin 10 deflects the resilient pawl 19, until the pin 10 is trapped behind sprag 26 at position b. In this position the slidable sleeve 5 is trapped on the needle hubs 7, 8 and cannot be removed without applying considerable force. This is the position of the components as supplied to the end user, and the location of the locking ring 13 takes account of this. With the locking ring 13 removed, the slidable sleeve 5 is pushed further towards the syringe 2, and pin 10 again deflects the resilient pawl 19 until the pin 10 reaches position c. This distance i defines the initial displacement of the slidable sleeve 5, when starting the injection, and the tip of needle 3 may be level with the face 6 of slidable sleeve 5. The slidable sleeve 5 may now be moved towards the syringe until the pin 10 reaches the end wall 28 of the recess 27 at position d. This position defines the maximum displacement of slidable sleeve 5, and thus the maximum exposure of the needle 3. At any time the force acting on slidable sleeve 5 is removed, the slidable sleeve 5 will return in the direction of arrow Y until pin 10 reaches the position e. Pin 10 would also help prevent the removal of slidable sleeve 5, but additionally the tooth 21 is now proximate to face 22 on a cantilever arm 9, which prevents the removal of the slidable sleeve 5. In this final position, pawl 20 engages with face 16 of hub 7 and prevents the slidable sleeve 5 from being moved. It should be noted that with the present diagrammatic presentation of the safety needle, a small amount of rotational movement is necessary between the slidable sleeve 5 and hub 7 to permit the pin 10 to move from position c to position e, but the rotation is preferably negligible.

The detent mechanism is interchangeable between the slidable sleeve 5 and needle hub 7 if required. Also, the detent mechanism described hereinabove is but one of a number of such mechanisms, the main requirement being to permit the following sequence of operation: permit the slidable sleeve to be moved sufficiently so that the opening in the slidable sleeve is level with or just in front of the needle tip, at which position the detent must be activated so that if the displacing force on the slidable sleeve is removed, the slidable sleeve slides forward and locks, thus protecting the user from contact with the needle tip. Typically, the tip of the needle would be about 3 mm back from the face of the opening in the slidable sleeve at the start, and 1 mm back from the face when the detent is activated.

Fig. 7 shows the device 1 as previously described, except that in this embodiment the needle 3 is bonded into the outlet end of the syringe barrel 29. The needle is free to pass through the needle hub 7, and the cone 9 is adapted at 31 to snap-fit over the projection 30. The form of projection 30 is produced as a result of rolling the glass onto a mandrel, whereby the excess glass is forced in to the shape as shown. Alternatively, a more defined snap-fitting termination may be formed, the object being to make the device 1 difficult to remove after assembling it to the syringe barrel.

Fig. 8 shows a safety needle 1 in which the needle hub 7 is integral with the barrel of the syringe 2. The slidable sleeve 5 is then attached to the needle hub 7 in the manner as described hereinbelow. The material of the needle hub 7, which also constitutes the barrel of the syringe, would, of course, have to be made from a drug-compatible material.

As mentioned hereinabove, Fig. 8 also shows the separate feature of the projection 32 on the surface 18 of the needle hub 7 which may be used to deflect the slidable sleeve 5 thereby generating the restoring force. This embodiment, i.e. incorporating the projection 32, provides a highly non-linear return rate since the length of the slidable sleeve 5 is effectively reduced as the slidable sleeve 5 and the needle hub 7 are slid together, thereby increasing the stiffness of the slidable sleeve 5.

As an alternative to the tapered outer surface of the needle hub 7, the slidable sleeve has at least one cantilever arm which engages a helical track in the outer surface of the needle hub such that, in use, as the needle is inserted into a patient, the at least one cantilever arm is displaced radially by the helical track in the outer surface of the needle hub thereby generating the restoring force. Thus, as the slidable sleeve 5 is caused to move towards the receiving end of the needle hub 7, one or more cantilever arms 9 are forced to follow the direction of the helical tracks. Since the cantilever arms 9 are resilient, a restoring force will be generated.

As an alternative to cantilever arms, the receiving end of the slidable sleeve itself may have elastic properties such that, in use, as the needle is inserted into a patient, the restoring force is generated within the slidable sleeve. By elastic properties the applicant means that the restoring force is generated within a radially continuous slidable sleeve, i.e. a sleeve without cantilever arms. The elastic properties may be achieved by using an elastic material, such as an elastomeric polymer. Alternatively, the receiving end of the slidable sleeve may be concertinaed, with the ridges, of course, running parallel to the hollow needle. The elastic properties could also be generated using a circumambient spring attached to the slidable sleeve.

As a further alternative, instead of the restoring force being generated in the slidable sleeve 5 itself, the safety needle may incorporate an alternative, or additional, restoring mechanism, such as a helical spring. Such safety needles are exemplified in US 4,911,693, US 4,813,940 and US 5,104,384.

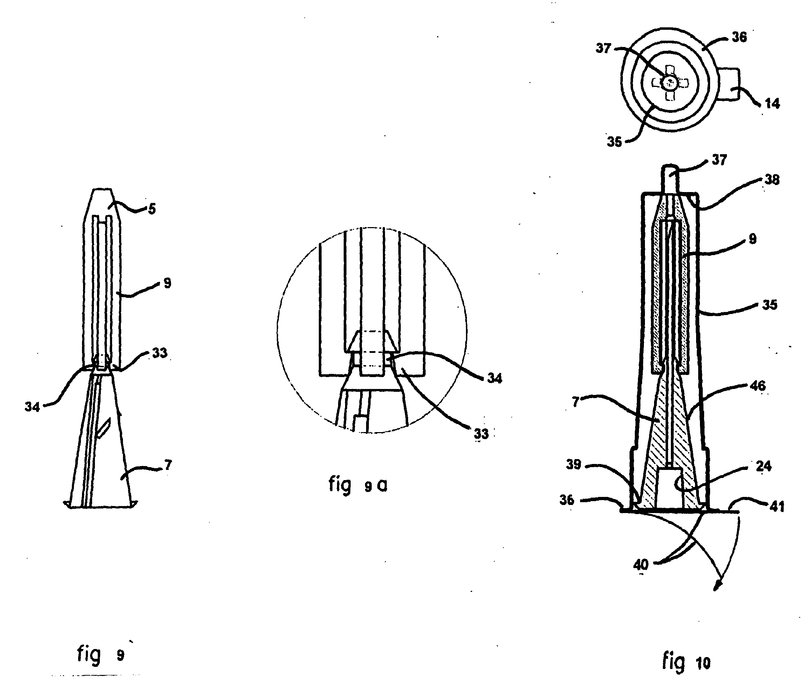

Figs. 9-14a show an embodiment of the present invention in which the needle hub and the slidable sleeve are adapted to allow the slidable sleeve to be retracted into and held at an intermediate position between the extended position and the retracted position such that, in use, the tip of the needle 2 projects partially from the slidable sleeve, that is the needle bevel is exposed. In this intermediate position the locking mechanism is not engaged and hence the slidable sleeve may be retracted further into the (fully) retracted position as it is inserted into the patient. The advantages of this arrangement are that exposing the tip of the needle partially allows the user to position the needle more precisely on, for example, the patient's skin, and also facilitates the aspiration of trapped air and excess drug. The use of the pack prevents needle stick injuries when the safety needle is in the intermediate position.

Fig. 9 shows the device as assembled by the manufacturer, and comprises a needle hub 7, being of conical or other tapering form. Slidable sleeve 5 has cantilever arms 9 attached or integral, the arms terminating with a projection 33, which engages with undercut 34 of the needle hub 7, shown in greater detail in Fig. 9a. In the position shown, there is little load on the spring cantilever arms 9, but sufficient to hold the components together. Referring to Fig. 10, this is a centre-line cross-section through the safety needle assembled into its pack 35. Pack 35 is releasably mounted on the needle hub and slidable sleeve. The pack 35 preferably tubular and also preferably made from a deep-drawn vacuum formed plastics material. The pack 35 is shown with a flange 36 and open at the receiving end of the needle hub 7, and closed at the injection end by the extension 37. The inner face of shoulder 38 rests on the end face of the slidable sleeve 5. The needle hub 7 has one or more projections 39, which provide a light frictional retaining force on the inside of the pack 35 to prevent the safety needle from falling out. The safety needle may be further retained inside the pack 35 by a releasable (peel-off) membrane 40, which is preferably gas permeable. The membrane 40 is bonded to flange 36, and may be made from a porous material such as Tyvek® which is spun-bonded high-density polyethylene available from DuPont and which is used extensively in pharmaceutical packaging to permit a sterilising gas, such as ethylene oxide, to penetrate the pack whilst preventing ingress of bacteria during storage. Other peelable materials may be used according to the sterilisation process used. The membrane 40 may have a tag 41 to assist removal. The needle hub 7 has a syringe adaptor 24 which may be configured to suit the common Luer taper or threaded Luer lock syringe nozzles.

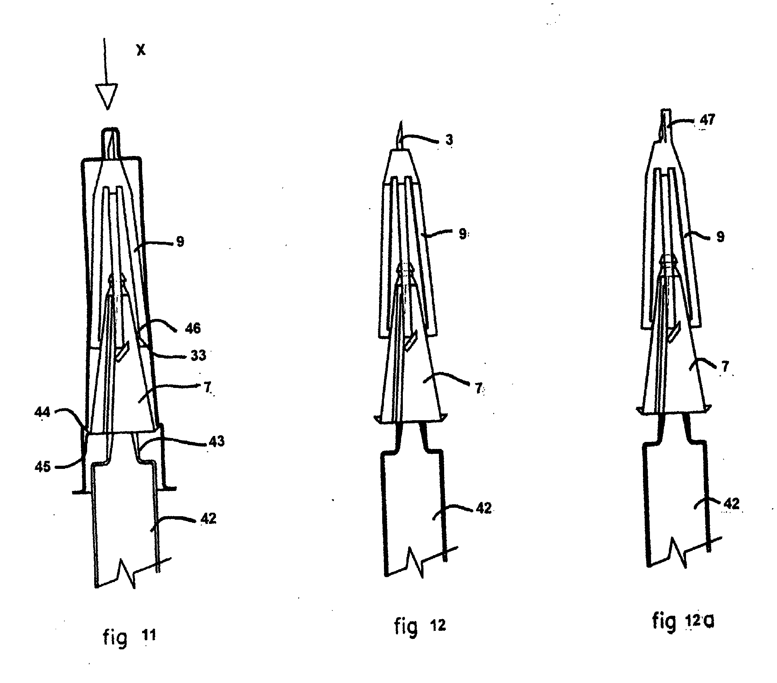

Referring to Fig. 11, to assemble the safety needle to a pre-filled syringe 42, the user removes the peelable membrane 40 by pulling on tag 41. Holding the pack 35, the user pushes the adaptor 24 of the needle hub 7 in the direction of the arrow X onto the syringe connector 43. This causes the inner face of the shoulders 38 to press against face of slidable sleeve 5 which moves towards the syringe. The injection end of the pack 35 has an extended portion which is capable of housing the tip of the needle 3 in the intermediate position and the shoulders 38 of the extended portion abut against the injection end of the slidable sleeve thereby causing the slidable sleeve 5 to move in to the intermediate position when the pack 35 is caused to move towards the receiving end of the needle hub 7. At the same time, the cantilever arms 9 are forced outwards as they travel up the surface of the needle hub 7, until the small shoulder 44 on the slidable sleeve 5 reaches the projection 45 on the needle hub 7, thus preventing further movement. At or about this point, at least one of the projections 33 snaps over a catch 46 on the needle hub 7. The cantilever arms 9 are now loaded radially, and exerting a resultant force urging the slidable sleeve 5 off the needle hub 7. The pack 35 may now be removed, and catch 46 prevents the slidable sleeve 5 from coming off the needle hub 7 through the resultant force of the cantilever arms 9. With pack 35 removed, the safety needle will appear as shown in Fig. 12, and is ready for use. The tip needle 3 is thus partially exposed, i.e. the bevel of the needle may be seen by the user projecting from the slidable sleeve 5, and the user may aspirate trapped air and excess drug. Fig. 12a shows the safety needle ready for use, as in Fig. 12, but with an extension 47 on face of slidable sleeve 5 extended to shroud partly the tip of the needle 3, which will afford more protection, but make aspiration of air and excess drug slightly more difficult.

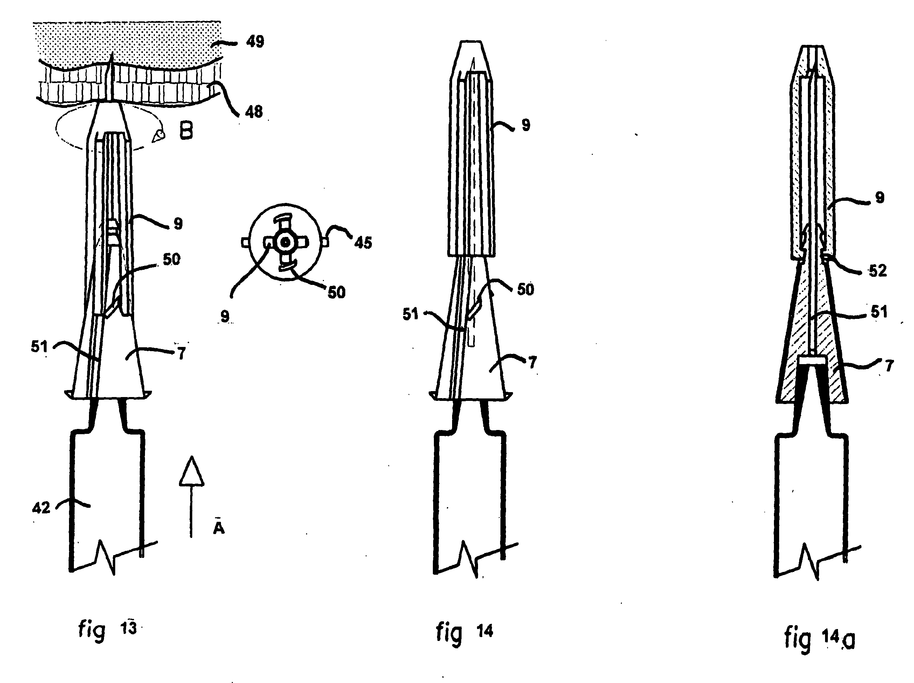

Referring to Fig. 13, the user pushes the needle 3 in the direction of arrow A through the patient's epidermis 48 and into the subcutaneous tissue 49, which brings the face of the slidable sleeve 5 into contact with the stratum corneum of the patient's epidermis 48. Further movement in the direction of arrow A pushes the slidable sleeve 5 towards the syringe 42, and thus the cantilever arms 9 are forced further outwards by the conical surface of the needle hub 7. At the same time, the end of at least one cantilever arm 9 is forced against a cam 50 which causes the slidable sleeve 5 and cantilever arms 9 to rotate in the direction of arrow B, until the end of cantilever arm 9 drops into the groove 51. Groove 51 has a slope towards the needle, which maintains the resultant force of the cantilever arm 9. At this point, (which represents only a millimetre or two of movement of the slidable sleeve 5), if the safety needle is withdrawn from the patient, the resultant force of the cantilever arms 9 urges the slidable sleeve 5 towards the tip of the needle 3 until the tip of the needle 3 is shielded by the slidable sleeve 5. At the end of travel of the projection 33 and the cantilever arm 9, the projection 33 on cantilever arm 9, which is still sliding in the groove 51, drops into the hole 52. This locks the slidable sleeve 5 in position and prevents the slidable sleeve 5 being pushed back towards the syringe 42, or being pulled off the needle hub 7. This position is shown in Figs. 14 and 14a, the latter being a section through the centre-line, and the safety needle rotated to show the grooves 51 and holes 52, with the projections 33 on cantilever arms 9 located within the holes 52. Although the lockably engaging projection 33 and hole 52 are shown with reference to cantilever arms 9, any slidable sleeve 5 may be locked into position using one or more projections 33 and corresponding one or more holes 52. Such an arrangement results in a less complex, and hence less costly, safety needle accessory and avoids introducing opposing frictional and/or detent forces which result from an integral but independent locking mechanism. An advantage of the projection 33/hole 52 mechanism is that the locking mechanism provides substantially no resistance against the restoring force as the slidable sleeve 5 moves from the retracted position to the extended position. When the slidable sleeve 5 reaches the extended position the locking mechanism engages which then resists the restoring force.

The protective pack 35 confers safe storage and handling advantages, allows the safety needle to be assembled to a syringe without risking premature operation of the safety mechanism, and does not add to the overall cost of the device, since it is similar to the vacuum-formed packs already in use for needles and syringes. For the user, the operation of the safety needle is practically identical to the use of a standard needle.

It is preferable that the coefficient of friction between the slidable sleeve 5 and the needle hub 7 is low, so that the resultant biasing force to return the slidable sleeve is not compromised by "stiction", or so high that the force required on the patient's skin to deflect the slidable sleeve 5 is excessive. This may be achieved by careful selection of materials. Such materials are known in the art, for example, the needle hub could be made from a high-density polyethylene or similar drug-compatible plastics material, and the slidable sleeve from an inexpensive plastics material such as polycarbonate, polystyrene, polyester or PVC. A more expensive, highly creep-resistant plastics material, for example polyphenylene sulfone, could also be used. As an alternative, the slidable sleeve, or just the at least one cantilever arm, may be made from metal, preferably stainless steel. The metal would be fabricated sufficiently thinly to provide the required elastic properties. If necessary, a lubricant may be used, or a lubricant may be incorporated with the polymers. Generally the materials should be suitable for sterilisation by gamma radiation, but it is possible to select materials compatible with sterilisation by steam or other gas such as ethylene oxide.

In a preferred embodiment, the slidable sleeve, prior to use, is not under any substantial load. Any substantial load indicates a load which is sufficient to cause the material of the slidable sleeve to undergo creepage during storage at ambient temperature.

As previously mentioned hereinabove, the syringe may be supplied empty or pre-filled. When a pre-filled syringe is used, the syringe is preferably sealed using a sealing cap or plug to prevent evaporation or loss of the drug, excipient, carrier and/or diluent by, for example, thermal expansion.

As well as application to a syringe, the same safety needle accessory described herein could form the basis of a intravenous giving set, so that the insertion of the needle into the patient's vein is simple and safe. Indeed, the safety needle accessory of the present invention may be used with any suitable injection device.

Other modifications of the present invention will be apparent to those skilled in the art.

## Claims

1. A safety needle (1) comprising a hollow needle (3) having a tip, a needle hub (7) surrounding the hollow needle, a slidable sleeve (5) slidably mounted on the needle hub and a pack (35) surrounding the hollow needle, needle hub and slidable sleeve,
the needle hub (7), the slidable sleeve (5) and the pack (35) each having a receiving end which is distal to the tip of the needle (3) and an injection end which is proximal to the tip of the needle (3),
wherein the receiving end of the needle hub (7) is suitable for connection to an injection device and the slidable sleeve (5) is adapted to slide in the direction of the length of the needle (3) between an extended position in which the tip of the needle (3) is located inside the slidable sleeve (5) and a retracted position in which the tip of the needle (3) projects from the slidable sleeve (5), via an intermediate position between the extended position and the retracted position in which the tip of the needle (3) projects partially from the slidable sleeve (5), such that, in use, the slidable sleeve (5) is moved into the intermediate position for injection into a patient and then as the needle (3) is inserted into a patient, the slidable sleeve (5) is caused to move into the retracted position,
wherein the needle hub (7) has an outer surface (18) which is adapted to deflect the slidable sleeve (5) as, in use, the needle (3) is inserted into the patient, and the slidable sleeve (5) bears resiliently on the outer surface (18) of the needle hub (7) such that, in use, a restoring force is generated within the slidable sleeve (5) as the slidable sleeve (5) is caused to move towards the receiving end of the needle hub (7) and into the retracted position, such that on removal of the needle (3) from the patient the restoring force causes the slidable sleeve (5) to move towards the injection end of the needle hub (7) and into the extended position, the safety needle (1) further comprising a locking mechanism capable of retaining the slidable sleeve in the extended position after removal of the needle from the patient, and wherein the pack (35) is releasably mounted on the needle hub (7) and slidable sleeve (5) such that the injection end of the pack (35) covers at least the injection end of needle (3) and the receiving end of the pack (35) has an open portion to expose the receiving end of the needle hub (7), and by causing the pack (35) to be moved in a direction towards the receiving end of the needle hub (7), the pack (35) engages with the slidable sleeve (5) which is retracted from the extended position to the intermediate position.

2. A safety needle as claimed in claim 1, wherein the open portion is covered by a releasable membrane (40).

3. A safety needle as claimed in claim 2, wherein the releasable membrane (40) is gas permeable.

4. A safety needle as claimed in any preceding claim, wherein the injection end of the pack (35) has an extended portion (37) which is capable of housing the needle tip in the intermediate position and shoulders (38) of the extended portion abut against the injection end of the slidable sleeve (5) thereby causing the slidable sleeve (5) to move in to the intermediate position when the pack (35) is caused to move towards the receiving end of the needle hub (7).

5. A safety needle as claimed in any preceding claim, wherein the needle hub (7) has one or more projections (39) which abut against one or more shoulders on the inside of the pack (35) when the slidable sleeve (5) is in the intermediate position.

6. A safety needle as claimed in any preceding claim, wherein the pack (35) is tubular.

7. A safety needle as claimed in any preceding claim, wherein the pack (35) is made from a vacuum-formed plastics material.

8. A safety needle as claimed in claim 1, wherein at least part of the receiving end of the needle hub (7) has a tapered outer surface (18) which tapers towards the injection end such that, in use, as the needle (3) is inserted into a patient, the slidable sleeve (5) is displaced outwards by the tapered outer surface (18) of the needle hub (7) thereby generating the restoring force.

9. A safety needle pack as claimed in claim 8, wherein the outer surface (18) of the receiving end of the needle hub (7) has a substantially conical shape which tapers towards the injection and.

10. A safety needle as claimed in any of claims 1 to 9, wherein the slidable sleeve (5) has at least one cantilever arm (9) which bears resiliently on the tapered outer surface (18) of the needle hub (7) and, in use, as the needle (3) is inserted into a patient, the at least one cantilever arm (9) is displaced outwards by the tapered outer surface (18) of the needle hub (7) thereby generating the restoring force.

11. A safety needle as claimed in any of claims 1 to 10, wherein the slidable sleeve (5) has at least one cantilever arm (9) which engages a helical track in the outer surface (18) of the needle hub (7) such that, in use, as the needle (3) is inserted into a patient, the at least one cantilever arm (9) is displayed radially by the helical track in the outer surface (18) of the needle hub (7) thereby generating the restoring force.

12. A safety needle as claimed in any of claims 1 to 11, wherein the safety needle has 2 to 6 cantilever arms (9).

13. A safety needle as claimed in claim 12, wherein the safety needle has 4 cantilever arms.

14. A safety needle as claimed in any of claims 1 to 9, wherein the receiving end of the slidable sleeve (5) has clastic properties and, in use, as the needle (3) is inserted into a patient, the restoring force is generated within the slidable sleeve (5).

15. A safety needle as claimed in any of claims 1 to 9, wherein the receiving end of the slidable sleeve (5) is concertinaed.

16. A safety needle as claimed in any preceding claim, wherein the locking mechanism provides substantially no resistance against the restoring force as the slidable sleeve (5) moves from the retracted position to the extended position.

17. A safety needle as claimed in any preceding claim, wherein the slidable sleeve (5) has a first extended position where the slidable sleeve (5) is able to be moved towards the receiving end of the needle hub (7) and a second extended position where the slidable sleeve (5) is in a locked position.

18. A safety needle as claimed in claim 17, wherein the locking mechanism comprises a latching pawl (20) on the inside surface of the slidable sleeve capable of latching to a shoulder (16) on the outside surface of the needle hub (7) when the slidable sleeve is in the second extended position.

19. A safety needle as claimed in claim 17 or 18, wherein an inside surface of the slidable sleeve (5) has a pin (10) which engages a sprag (26) and a resilient pawl (19) attached to the needle hub (7) thereby holding the slidable sleeve (5) in the first extended position and, in use, allowing the slidable sleeve (5) to move into the second extended position.

20. A safety needle as claimed in any of claims 1 to 17, wherein the locking mechanism comprises one or more projections (33) on the slidable sleeve (5) and a corresponding one or more holes (52) on the needle hub (7) such that in the extended position after removal from the patient the one or more projections (33) lockable engage the corresponding one or more holes (52).

21. A safety needle as claimed in any preceding claim, wherein the safety needle pack (35) is constructed from materials capable of being sterilised with gamma irradiation and/or ethylene oxide.

22. A safety needle as claimed in any preceding claim, further comprising a helical spring which, in use, generates the restoring force as the slidable sleeve (5) is caused to move towards the receiving end of the needle hub (7) and into the retracted position.

23. A safety needle as claimed in any preceding claim wherein, the slidable sleeve (5), prior to use, is not under any substantial load.

24. A method for priming an injection device comprising the steps of inserting an injection device in to the receiving end of the needle hub (7) of the safety needle as claimed in any preceding claim, moving the pack (35) towards the injection device such that the slidable sleeve (5) moves in to the intermediate position, and removing the pack (35).

25. A safety needle as claimed in claim 20, wherein the one or more holes (52) on the needle hub (7) is a circumferentially continuous hole.

26. A safety needle as claimed in claim 1, wherein the needle hub (7) is adapted to releasably retain the slidable sleeve (5) in an intermediate position between the extended position and the retracted position.

27. A safety needle as claimed in claim 1, wherein the needle tip is partially shrouded in the intermediate position such that a needle bevel is visible.

## Patentansprüche

1. Sicherheitskantile (1), bestehend aus einer Hohlkanüle (3), die eine Spitze, einen die Hohlkanäle umschließenden Kanülenansatz (7), eine verschiebbar am Kanülenansatz montierte Gleithülse (5) sowie eine die Hohlkanüle, den Kanülenansatz und die Gleithülse umschließende Ummantelung (35) aufweist,
wobei der Kanülenansatz (7), die Gleithülse (5) und die Ummantelung (35) jeweils eine Eingangsseite aufweisen, die distal zur Spitze der Kanüle (3) angeordnet ist, und eine Injektionsseite, die proximal zur Spitze der Kanüle (3) angeordnet ist,
wobei die Eingangsseite des Kanülenansatzes (7) zum Verbinden mit einer Injektionsspritze geeignet ist und die Gleithülse (5) so ausgelegt ist, dass sie in Längsrichtung der Kanüle (3) zwischen einer ausgefahrenen Position, in der sich die Spitze der Kanüle (3) innerhalb der Gleithülse (5) befindet, und einer eingefahrenen Position, in der die Spitze der Kanüle (3) über die Gleithülse (5) hinausragt, über eine Mittelstellung zwischen der ausgefahrenen und der eingefahrenen Position, in der die Spitze der Kanüle (3) teilweise über die Gleithülse (5) hinausragt, gleiten kann, so dass bei der Anwendung die Gleithülse (5) zur Injektion in einen Patienten in die Mittelstellung bewegt wird und danach die Gleithülse (5) beim Einführen der Kanüle (3) in den Patienten in die eingefahrene Position bewegt wird,
wobei der Kanülenansatz (7) eine Außenfläche (18) aufweist, die so ausgelegt ist, dass die Gleithülse (5) abgelenkt wird, wenn die Kanüle (3) bei der Anwendung in den Patienten eingeführt wird, und die Gleithülse (5) elastisch an der Außenfläche (18) des Kanülenansatzes (7) abgestützt wird, so dass bei der Anwendung eine Rückholkraft innerhalb der Gleithülse (5) erzeugt wird, wenn die Bewegung der Gleithülse (5) in Richtung der Eingangsseite des Kanülenansatzes (7) und in die eingefahrene Position bewirkt wird, so dass bei Entfernen der Kanüle (3) vom Patienten die Rückholkraft bewirkt, dass die Gleithülse (5) sich in Richtung der Injektionsseite des Kanülenansatzes (7) und in die ausgefahrene Position bewegt; weiterhin weist die Sicherheitskanüle (1) einen Verriegelungsmechanismus auf, der in der Lage ist, die Gleithülse nach dem Entfernen der Kanüle vom Patienten in der ausgefahrenen Position zu halten, wobei die Ummantelung (35) lösbar auf dem Kanülenansatz (7) und der Gleithülse (5) angebracht ist, so dass die Injektionsseite der Ummantelung (35) mindestens die Injektionsseite der Kanüle (3) bedeckt, und die Eingangsseite der Ummantelung (35) einen offenen Teil aufweist, der die Eingangsseite des Kanülenansatzes (7) freilegt, und bei der somit ausgelösten Bewegung der Ummantelung (35) in Richtung der Eingangsseite des Kanülenansatzes (7) die Ummantelung (35) in die Gleithülse (5) eingreift, die aus der ausgefahrenen Position in die Mittelstellung eingefahren wird.

2. Sicherheitskanüle nach Anspruch 1, wobei der offene Teil mit einer ablösbaren Membran (40) bedeckt ist.

3. Sicherheitskanüle nach Anspruch 2, wobei die ablösbare Membran (40) gasdurchlässig ist.

4. Sicherheitskanüle nach einem der vorhergehenden Ansprüche, wobei die Injektionsseite der Ummantelung (35) einen verlängerten Teil aufweist (37), der die Kanülenspitze in der Mittelstellung aufnehmen kann, sowie Ansätze (38) des verlängerten Teils, die an der Injektionsseite der Gleithülse (5) anliegen und dabei bewirken, dass die Gleithülse (5) in die Mittelstellung bewegt wird, wenn die Ummantelung (35) in Richtung der Eingangsseite des Kanülenansatzes bewegt wird (7).

5. Sicherheitskanüle nach einem der vorhergehenden Ansprüche, wobei der Kanülenansatz (7) einen oder mehrere Vorsprünge aufweist (39), die an einem oder mehreren Ansätzen im Inneren der Ummantelung (35) anliegen, wenn sich die Gleithülse (5) in der Mittelstellung befindet.

6. Sicherheitskanüle nach einem der vorhergehenden Ansprüche, wobei die Ummantelung (35) rohrförmig ausgeführt ist.

7. Sicherheitskanüle nach einem der vorhergehenden Ansprüche, wobei die Ummantelung (35) aus vakuumgeformtem Kunststoff besteht.

8. Sicherheitskanüle nach Anspruch 1, wobei mindestens ein Teil der Eingangsseite des Kanülenansatzes (7) eine konische Außenfläche (18) aufweist, die sich in Richtung der Injektionsseite verjüngt, so dass bei der Anwendung bei Einführung der Kanüle (3) in den Patienten die Gleithülse (5) durch die konische Außenfläche (18) des Kanülenansatzes (7) nach außen bewegt wird und dabei die Rückholkraft erzeugt.

9. Sicherheitskanülen-Ummantelung nach Anspruch 8, wobei die Außenfläche (18) der Eingangsseite des Kanülenansatzes (7) eine im Wesentlichen konische Form aufweist, die sich in Richtung der Injektionsseite verjüngt.

10. Sicherheitskanüle nach einem der Ansprüche 1 bis 9, wobei die Gleithülse (5) mindestens eine Auskragung (9) aufweist, die elastisch auf der konischen Außenfläche (18) des Kanülenansatzes (7) aufliegt, und bei der Anwendung bei Einführung der Kanüle (3) in den Patienten die mindestens eine Auskragung (9) durch die konische Außenfläche (18) des Kanülenansatzes (7) nach außen bewegt wird und dabei die Rückholkraft erzeugt.

11. Sicherheitskanüle nach einem der Ansprüche 1 bis 10, wobei die Gleithülse (5) mindestens eine Auskragung (9) aufweist, die in eine spiralförmige Führung in der Außenfläche (18) des Kanülenansatzes (7) eingreift, so dass bei der Anwendung bei Einführung der Kanüle (3) in den Patienten die mindestens eine Auskragung (9) durch die spiralförmige Führung in der Außenfläche (18) des Kanülenansatzes (7) radial bewegt wird und dabei die Rückholkraft erzeugt.

12. Sicherheitskanüle nach einem der Ansprüche 1 bis 11, wobei die Sicherheitskanüle 2 bis 6 Auskragungen (9) aufweist.

13. Sicherheitskanüle nach Anspruch 12, wobei die Sicherheitskanüle 4 Auskragungen aufweist.

14. Sicherheitskanüle nach einem der Ansprüche 1 bis 9, wobei die Eingangsseite der Gleithülse (5) elastische Eigenschaften aufweist und bei der Anwendung bei Einführung der Kanüle (3) in den Patienten die Rückholkraft innerhalb der Gleithülse (5) erzeugt wird.

15. Sicherheitskanüle nach einem der Ansprüche 1 bis 9, wobei die Eingangsseite der Gleithülse (5) faltenbalgartig funktioniert.

16. Sicherheitskanüle nach einem der vorhergehenden Ansprüche, wobei der Verriegelungsmechanismus der Rückholkraft im Wesentlichen keinen Widerstand bietet, wenn sich die Gleithülse (5) von der eingefahrenen Position in die ausgefahrene Position bewegt.

17. Sicherheitskanüle nach einem der vorhergehenden Ansprüche, wobei die Gleithülse (5) eine erste ausgefahrene Position aufweist, in der die Gleithülse (5) in Richtung der Eingangsseite des Kanülenansatzes (7) bewegt werden kann, sowie eine zweite ausgefahrene Position, in der die Gleithülse (5) sich in einer verriegelten Position befindet.

18. Sicherheitskanüle nach Anspruch 17, wobei der Verriegelungsmechanismus eine Verriegelungsraste (20) an der Innenfläche der Gleithülse aufweist, die in einen Ansatz (16) an der Außenfläche des Kanülenansatzes (7) einrasten kann, wenn sich die Gleithülse in der zweiten ausgefahrenen Position befindet.

19. Sicherheitskanüle nach Anspruch 17 oder 18, wobei eine Innenfläche der Gleithülse (5) einen Stift (10) aufweist, der in einen Keil (26) und eine elastische Raste (19) eingreift, die am Kanülenansatz (7) angebracht sind, und dabei die Gleithülse (5) in der ersten ausgefahrenen Position hält und bei der Anwendung die Bewegung der Gleithülse (5) in die zweite Position ermöglicht.

20. Sicherheitskanüle nach einem der Ansprüche 1 bis 17, wobei der Verriegelungsmechanismus einen oder mehrere Vorsprünge (33) an der Gleithülse (5) und entsprechend eine oder mehrere Vertiefungen (52) am Kanülenansatz (7) aufweist, so dass nach dem Abnehmen vom Patienten in ausgefahrener Position der eine Vorsprung bzw. mehrere Vorsprünge (33) blockierbar in die entsprechende Vertiefung bzw. in mehrere Vertiefungen (52) eingreift bzw. eingreifen.

21. Sicherheitskanüle nach einem der vorhergehenden Ansprüche, wobei die Ummantelung (35) der Sicherheitskanüle aus Material hergestellt ist, das mit Gamrnabestrahlung und/oder Ethylenoxid sterilisiert werden kann.

22. Sicherheitskanüle nach einem der vorhergehenden Ansprüche, die ferner eine Schraubenfeder umfasst, die bei der Anwendung die Rückholkraft erzeugt, während die Gleithülse (5) zur Eingangsseite des Kanülenansatzes (7) und in die eingefahrene Position bewegt wird.

23. Sicherheitskanüle nach einem der vorhergehenden Ansprüche, wobei die Gleithülse (5) vor der Anwendung nicht erheblich belastet ist.

24. Ein Verfahren zum Füllen einer Injektionsspritze, das die folgenden Schritte umfasst: Einführen einer Injektionsspritze in die Eingangsseite des Kanülenansatzes (7) der Sicherheitskanüle nach einem der vorhergehenden Ansprüche, wobei der Kanülenansatz (35) zur Injektionsspritze bewegt wird, so dass die Gleithülse (5) sich in die Mittelstellung bewegt, und Abnehmen der Ummantelung (35).

25. Sicherheitskanüle nach Anspruch 20, wobei die eine Vertiefung oder mehrere Vertiefungen (52) am Kanülenansatz (7) als umlaufende durchgehende Vertiefung ausgeführt ist bzw. sind.

26. Sicherheitskanüle nach Anspruch 1, wobei der Kanülenansatz so ausgeführt ist, dass er die Gleithülse (5) lösbar in einer Mittelstellung zwischen der ausgefahrenen und der eingefahrenen Position hält.

27. Sicherheitskanüle nach Anspruch 1, wobei die Kanülenspitze in Mittelstellung teilweise verdeckt ist, so dass eine Abschrägung der Kanüle sichtbar ist.

## Revendications

1. Aiguille de sécurité (1) comprenant une aiguille creuse (3) ayant une pointe, un pavillon d'aiguille (7) qui entoure l'aiguille creuse, un manchon coulissant (5) monté avec faculté de coulissement sur le pavillon d'aiguille et un fourreau (35) qui entoure l'aiguille creuse, le pavillon d'aiguille et le manchon coulissant ;
le papillon d'aiguille (7), le manchon coulissant (5) et le fourreau (35) ayant chacun une extrémité réceptrice qui est distale de la pointe de l'aiguille (3) et une extrémité d'injection qui est proximale à la pointe de l'aiguille (3) ;
dans laquelle l'extrémité de réception du pavillon d'aiguille (7) est adaptée pour être raccordée à un dispositif d'injection et le manchon coulissant (5) est adapté pour coulisser dans le sens de la longueur de l'aiguille (3) entre une position déployée dans laquelle la pointe de l'aiguille (3) est située à l'intérieur du manchon coulissant (5) et une position rétractée dans laquelle la pointe de l'aiguille (3) dépasse du manchon coulissant (5), par une position intermédiaire entre la position déployée et la position rétractée dans laquelle la pointe de l'aiguille (3) dépasse partiellement du manchon coulissant (5), de telle sorte que, durant l'utilisation, le manchon coulissant (5) soit déplacé à l'intérieur de la position intermédiaire pour l'injection dans un patient puis quand l'aiguille (3) est insérée dans un patient, le manchon coulissant (5) soit amené à se déplacer dans la position rétractée,
dans laquelle le pavillon d'aiguille (7) comporte une surface externe (18) qui est adaptée pour dévier le manchon coulissant (5) lorsque, durant l'utilisation, l'aiguille (3) est insérée dans le patient, et le manchon coulissant (5) repose de façon élastique sur la surface externe (18) du pavillon d'aiguille (7) de telle sorte que, durant l'utilisation, une force de rétablissement soit générée à l'intérieur du manchon coulissant (5) lorsque le manchon coulissant (5) est amené à se déplacer vers l'extrémité réceptrice du pavillon d'aiguille (7) et dans la position rétractée, de telle sorte qu'au retrait de l'aiguille (3) hors du patient la force de rétablissement amène le manchon coulissant (5) à se déplacer vers l'extrémité d'injection du pavillon d'aiguille (7) et dans la position déployée, l'aiguille de sécurité (1) comprenant en outre un mécanisme de verrouillage capable de retenir le manchon coulissant dans la position déployée après le retrait de l'aiguille hors du patient, et dans laquelle le fourreau (35) est monté de façon détachable sur le pavillon d'aiguille (7) et le manchon coulissant (5) de telle sorte que l'extrémité d'injection du fourreau (35) couvre au moins l'extrémité d'injection de l'aiguille (3) et l'extrémité de réception du fourreau (35) ait une partie ouverte pour exposer l'extrémité réceptrice du pavillon d'aiguille (7), et en amenant le fourreau (35) à être déplacé dans un sens vers l'extrémité de réception du pavillon d'aiguille (7), le fourreau (35) s'engrène avec le manchon coulissant (5) qui est rétracté de la position déployée à la position intermédiaire.

2. Aiguille de sécurité selon la revendication 1, dans laquelle la partie ouverte est couverte par une membrane détachable (40).

3. Aiguille de sécurité selon la revendication 2, dans laquelle la membrane détachable (40) est perméable au gaz.

4. Aiguille de sécurité selon l'une quelconque des revendications précédentes, dans laquelle l'extrémité d'injection du fourreau (35) a une partie déployée (37) qui est capable de loger la pointe d'aiguille dans la position intermédiaire et des épaulements (38) de la partie déployée buttent contre l'extrémité d'injection du manchon coulissant (5) amenant ainsi le manchon coulissant (5) à se déplacer dans la position intermédiaire quand le fourreau (35) est amené à se déplacer vers l'extrémité réceptrice du pavillon d'aiguille (7).

5. Aiguille de sécurité selon l'une quelconque des revendications précédentes, dans laquelle le pavillon d'aiguille (7) comporte une ou plusieurs protubérances (39) qui buttent contre un ou plusieurs épaulements sur l'intérieur du fourreau (35) quand le manchon coulissant (5) est dans la position intermédiaire.

6. Aiguille de sécurité selon l'une quelconque des revendications précédentes, dans laquelle le fourreau (35) est tubulaire.

7. Aiguille de sécurité selon l'une quelconque des revendications précédentes, dans lequel le fourreau (35) est réalisé en un matériau plastique formé sous vide.

8. Aiguille de sécurité selon la revendication 1, dans laquelle au moins une partie de l'extrémité réceptrice du pavillon d'aiguille (7) comporte une surface externe conique (18) qui diminue progressivement vers l'extrémité d'injection de telle sorte que, durant l'utilisation, lorsque l'aiguille (3) est insérée dans un patient, le manchon coulissant (5) soit déplacé vers l'extérieur par la surface externe conique (18) du pavillon d'aiguille (7) générant ainsi la force de rétablissement.

9. Fourreau d'aiguille de sécurité selon la revendication 8, dans laquelle la surface externe (18) de l'extrémité réceptrice du pavillon d'aiguille (7) a une forme sensiblement conique qui diminue progressivement vers l'extrémité d'injection.

10. Aiguille de sécurité selon l'une quelconque des revendications 1 à 9, dans laquelle le manchon coulissant (5) comporte au moins un bras en porte-à-faux (9), lequel porte de façon élastique sur la surface externe conique (18) du pavillon d'aiguille (7) et, durant l'utilisation, quand l'aiguille (3) est insérée dans un patient, l'au moins un bras en porte-à-faux (9) soit déplacé vers l'extérieur par la surface externe conique (18) du pavillon d'aiguille (7) générant ainsi la force de rétablissement.

11. Aiguille de sécurité selon l'une quelconque des revendications 1 à 10, dans laquelle le manchon coulissant (5) comporte au moins un bras en porte-à-faux (9) qui s'engrène avec une piste hélicoïdale dans la surface externe (18) du pavillon d'aiguille (7) de telle sorte que, durant l'utilisation, lorsque l'aiguille (3) est insérée dans un patient, l'au moins un bras en porte-à-faux (9) soit déplacé radialement par la piste hélicoïdale dans la surface externe (18) du pavillon d'aiguille (7) générant ainsi la force de rétablissement.

12. Aiguille de sécurité selon l'une quelconque des revendications 1 à 11, l'aiguille de sécurité comportant de 2 à 6 bras en porte-à-faux (9).

13. Aiguille de sécurité selon la revendication 12, l'aiguille de sécurité comportant 4 bras en porte-à-faux.

14. Aiguille de sécurité selon l'une quelconque des revendications 1 à 9, dans laquelle l'extrémité réceptrice du manchon coulissant (5) a des propriétés élastiques et, durant l'utilisation, quand l'aiguille (3) est insérée dans un patient, la force de rétablissement est générée à l'intérieur du manchon coulissant (5).

15. Aiguille de sécurité selon l'une quelconque des revendications 1 à 9, dans laquelle l'extrémité réceptrice du manchon coulissant (5) est en accordéon.

16. Aiguille de sécurité selon l'une quelconque des revendications précédentes, dans laquelle le mécanisme de verrouillage n'offre sensiblement aucune résistance à la force de rétablissement quand le manchon coulissant (5) se déplace de la position rétractée à la position déployée.

17. Aiguille de sécurité selon l'une quelconque des revendications précédentes, dans laquelle le manchon coulissant (5) a une première position déployée où le manchon coulissant (5) peut être déplacé vers l'extrémité réceptrice du pavillon d'aiguille (7) et une seconde position déployée à laquelle le manchon coulissant (5) est en position verrouillée.

18. Aiguille de sécurité selon la revendication 17, dans laquelle le mécanisme de verrouillage comprend un cliquet de verrouillage (20) sur la surface interne du manchon coulissant capable de se verrouiller sur un épaulement (16) sur la surface extérieure du pavillon d'aiguille (7) quand le manchon coulissant est dans la seconde position déployée.

19. Aiguille de sécurité selon la revendication 17 ou 18, dans laquelle une surface interne du manchon coulissant (5) comporte une broche (10) qui s'engrène avec un galet (26) et un cliquet élastique (19) fixé au pavillon d'aiguille (7) retenant ainsi le manchon coulissant (5) à la première position déployée et, durant l'utilisation, permettant au manchon coulissant (5) de se déplacer dans la seconde position déployée.

20. Aiguille de sécurité selon l'une quelconque des revendications 1 à 17, dans laquelle le mécanisme de verrouillage comprend une ou plusieurs protubérances (33) sur le manchon coulissant (5) et un ou plusieurs trous correspondants (52) sur le pavillon d'aiguille (7) de telle sorte que dans la position déployée après le retrait hors du patient les une ou plusieurs protubérances (33) s'engrènent de manière verrouillable avec les un ou plusieurs trous correspondants (52).

21. Aiguille de sécurité selon l'une quelconque des revendications précédentes, dans laquelle le fourreau d'aiguille de sécurité (35) est construit en matériaux stérilisables par irradiation aux rayons gamma et/ou à l'oxyde d'éthylène.

22. Aiguille de sécurité selon l'une quelconque des revendications précédentes, comprenant en outre un ressort hélicoïdal qui, durant l'utilisation, génère la force de rétablissement quand le manchon coulissant (5) est amené à se déplacer vers l'extrémité réceptrice du pavillon d'aiguille (7) et dans la position rétractée.

23. Aiguille de sécurité selon l'une quelconque des revendications précédentes, dans laquelle le manchon coulissant (5), avant l'utilisation, ne subit aucune charge substantielle.

24. Procédé d'amorçage d'un dispositif d'injection comprenant les étapes d'insertion d'un dispositif d'injection dans l'extrémité réceptrice du pavillon d'aiguille (7) de l'aiguille de sécurité selon l'une quelconque des revendications précédentes, de déplacement du fourreau (35) vers le dispositif d'injection de telle sorte que le manchon coulissant (5) se déplace dans la position intermédiaire, et de retrait du fourreau (35).

25. Aiguille de sécurité selon la revendication 20, dans laquelle les un ou plusieurs trous (52) sur le pavillon d'aiguille (7) sont des trous de circonférence continue.

26. Aiguille de sécurité selon la revendication 1, dans laquelle le pavillon d'aiguille (7) est adapté pour retenir de manière détachable le manchon coulissant (5) dans une position intermédiaire entre la position déployée et la position rétractée.

27. Aiguille de sécurité selon la revendication 1, dans laquelle la pointe de l'aiguille est partiellement carénée dans la position intermédiaire de telle sorte qu'un biseau d'aiguille soit visible.
